# EUROPEAN PATENT APPLICATION

(11) **EP 2 985 603 A1**
(43) Date of publication of application: **17.02.2016**
(21) Application number: 14180879.0
(22) Date of filing: 13.08.2014
(51) Int. Cl.: G01N 33/543

(54) **Method for carrying out a ligand binding assay and device for carrying out such a method**

(71) Applicant: Yantai AusBio Laboratories Co., Ltd., Yeda Yantai Shandong (CN)
(72) Inventor: Wang, Zhaoqiang, Yantai 246006 (CN); Mann, Wolfgang, 95512 Hornungsreuth (DE)
(74) Representative: Ganahl, Bernhard

(57) **Abstract**

Method for carrying out a ligand binding assay in at least one reaction well, comprising the steps of filling the at least one reaction well with a sample liquid and overlaying the sample liquid with an oil film.

## Description

The invention relates to a method for carrying out a ligand binding assay. In particular, the present invention discloses a method for carrying out a ligand binding assay in a way that it can be automated easily.

Ligand binding assay is a term that refers to an assay on analytic procedure whose procedure or method relies on the binding of ligand molecules to receptors. Using the respective detection method (e.g. fluorescence, luminescence, chemiluminescence, etc.) the presence and extent of the formed ligand receptor complexes can be determined. This type of analytic test can be used to test for the presence of target molecules in a sample that are known to bind to the receptor.

Ligand binding assays provide a measure of the interactions that occur between two molecules such as protein bindings as well as the degree of affinity for which the reactants bind together. Essential aspects of binding assays include, but are not limited to, the concentration level of reactants of products maintaining the equilibrium constant of reactants throughout the assay and the reliability and validity of linked reactions.

Ligand binding assays can be classified in four different main types of assays: (i) radioactive ligand binding assays, (ii) non-radioactive ligand binding assays (e.g. fluorescence polarization (FP), fluorescence resonance energy transfer (FRET) or surface plasmon resonance (SPR)), (iii) liquid-phase ligand binding assays (e.g. real-time polymerase chain reaction (RT-qPCR) or immunoprecipitation) and (iv) solid-phase ligand binding assays (e.g. multiwell plate assays as for example bioassays and immunoassays, bead based ligand binding assays, on-column ligand binding assays or filter assays), which are well known to a person skilled in the art.

For multiwell plate assays multiwell plates, also called microplates, are used, which contain multiple reaction compartments (wells) incorporated into one container with the number of individual wells ranging from 6 to over 1536. These plates are convenient for handling necessary dosages and replicates. There is a wide range of plate types that have a standardized footprint, supporting equipment and measurement systems.

The main focus of the present invention is on the solid-phase ligand binding assay, especially immunoassays.

An immunoassay is a biochemical test that measures the presence or concentration of a target molecule in a sample through the use of antibodies and/or antigens. The molecule detected by the immunoassay is often referred to as an "analyte" and is in many cases a protein. Analytes in biological liquids such as serum or urine are frequently measured using immunoassays for medical (diagnostic) and research purposes.

Immunoassays come in many different formats and variations. Immunoassays may be run in multiple steps with reagents being added and washed away or separated at different points in the assay.

The use of a calibrator is often employed in immunoassays. Calibrators are solutions that are known to contain the analyte in question, and the concentration of that analyte is generally known. Comparison of an assay's response to a real sample against the assay's response produced by the calibrators makes it possible to interpret the signal strength in terms of the presence or concentration of analyte in the sample.

Immunoassays rely on the ability of an antibody to recognize and bind a specific target molecule. The particular molecule bound by an antibody is referred to as an antigen and the area on an antigen to which the antibody binds is called an epitope.

In some immunoassays, the analyte may be an antibody rather than an antigen.

In addition to the binding of an antibody to its antigen, the other key feature of all immunoassays is a means to produce a measurable signal in response to the binding. Most immunoassays involve chemically linking antibodies or antigens with some kind of detectable label. A large number of labels exist in modern immunoassays, and they allow for detection through different means. Many labels are detectable because they either emit radiation, produce a color change in a solution, fluoresce under light, or because they can be induced to emit light.

Therefore a variety of different labels are employed to allow for detection of antibodies and antigens in immunoassays. These are typically chemically linked or conjugated to the desired antibody or antigen.

One of the most popular labels to use in immunoassays is enzymes. Immunoassays which employ enzymes are referred to as enzyme-linked immunosorbent assays (ELISAs), or sometimes enzyme immunoassays (EIAs).

Enzymes used in ELISAs include horseradish peroxidase (HRP), alkaline phosphatase (AP) or Peroxidase. These enzymes allow for detection often because they produce an observable color change in the presence of certain reagents. In some cases these enzymes are exposed to reagents which cause them to produce light or chemiluminescence.

Another kind of labelling are radioactive isotopes, which can be incorporated into immunoassay reagents to produce a radioimmunoassay (RIA). Radioactivity emitted by bound antibody-antigen complexes can be easily detected using conventional methods.

Another approach to immunoassays involves combining real-time quantitative polymerase chain reaction (RT qPCR) and traditional immunoassay techniques. Called real-time immunoquantitative PCR (iqPCR) the label used in these assays is a DNA probe.

Fluorogenic reporters like phycoerythrine are used in a number of modern immunoassays. Protein microarrays are a type of immunoassay that often employ flourogenic reporters.

Some labels work on an electrochemiluminescent principle, in which the label emits detectable light in response to electrical current.

While some kind of label is generally employed in immunoassays, there are certain kinds of assays, which do not rely on labels, but instead employ detection methods that don't require the modification or labeling the components of the assay. Surface plasmon resonance is an example of technique that can detect binding between an unlabeled antibody and antigens. Another demonstrated labelless immunoassay involves measuring the change in resistance on an electrode as antigen binds to it.

Immunoassays can be run in a number of different formats. Generally, an immunoassay will fall into one of several categories depending on how it is run.

In a competitive, homogeneous immunoassay, unlabeled analyte in a sample competes with labelled analyte to bind to an antibody. The amount of labelled, unbound analyte is then measured. In theory, the more analyte in the sample, the more labelled analyte gets competed off and hence the amount of labelled, unbound analyte is proportional to the amount of analyte in the sample.

As in a competitive, homogeneous assay, in a competitive, heterogeneous immunoassay unlabeled analyte in a sample competes with labelled analyte to bind an antibody. In the heterogeneous assays the labelled, unbound analyte is separated or washed away, and the remaining labelled, bound analyte is measured.

In one-site, noncompetitive immunoassays the unknown analyte in the sample binds with labeled antibodies. The unbound, labeled antibodies are washed away, and the bound, labeled is measured, which is directly proportional to the amount of unknown analyte.

In two-site, noncompetitive immunoassays the analyte in the unknown sample is bound to the antibody site, then labeled antibody is bound to the analyte. The amount of labeled antibody on the site is then measured. It will be directly proportional to the concentration of the analyte because labeled antibody will not bind if the analyte is not present in the unknown sample. This type is also known as sandwich assay as the analyte is "sandwiched" between two antibodies.

As mentioned, in the field of solid-phase ligand binding assays ELISA can probably be regarded as the most popular one. ELISA is an analytic biochemistry assay that uses a solid-phase enzyme immunoassay to detect the presence of a substance, usually an antigen, in a liquid sample or wet sample. The ELISA is a specific and highly sensitive method for quantification of analytes in solution. It was first described in 1971 and has since gained the position as one of the most used methods in laboratory science. The ELISA method can be used in a variety of formats, e.g. for testing few samples as well as for high throughput screening.

There are different types of ELISA like the "indirect" ELISA (one-site, noncompetitive immunoassay), the sandwich ELISA (two-site, noncompetitive immunoassay) and the competitive ELISA (competitive, homogeneous or heterogeneous immunoassay) which individually can be adjusted depending on what kind of result is intended to be obtained.

A possible setup for an "indirect" ELISA consists of a known or unknown antigen samples coated to the reaction well, which will be bound by a labeled specific antibody. After washing away unbound reagents the labeled antibody allows an enzymatic reaction that converts a colorless substrate into a colored reaction product. The color change can be read directly in the reaction tray making data collection very easy.

A possible setup for a sandwich ELISA consists of a specific, preferably monoclonal, antibody coated to a reaction well, which is able to capture the protein of interest from a reaction sample. After addition of the sample the specific antibody on the plate will capture the protein. A second antibody used for detection binds a different epitope on the target molecule. The second antibody is separately labeled, e.g. with biotin. The separate labeling of the second antibody allows an enzymatic reaction that converts a colorless substrate into a colored reaction product. The color change can be read directly in the reaction tray making data collection very easy. The concentration of protein in the sample is determined by comparison with a standard curve with known protein concentrations.

A further solid-phase ligand binding assay, which is of interest for the present invention, is a so called bead based ligand binding assays. One possible setup for this kind of assay would be that magnetic beads are coated with an antibody specific for the target antigen. Then the same steps as for a sandwich ELISA are performed. The magnetic beads can be retained in the reaction well by magnetic interaction. By the addition of the respective reagent the antibody can again be removed from the bead.

Further, also so-called DNA capture assays can be performed according to method of the present invention. A possible way to perform such a DNA capture assay would be that an oligonucleotide probe is fixed to a solid surface. During hybridization labelled target molecules are captured according to the rules of base pairing. After a washing step the target molecules can be detected.

For a person skilled in the art it is obvious that the various assays described herein can vary in their experimental setups depending on the specific test to be carried out.

Furthermore, it is obvious to a person skilled in the art that the various assays that can be carried out according to the method of the present invention can be combined with each other or with other experimental processes. For example, a PCR reaction might be followed by a DNA capture assay.

A further example of procedures, which can be combined and carried out according to the present invention, is the so called immuno-PCR (iPCR), which is a ultrasensitive method to detect antigens or antibodies. One method to perform an iPCR is to use a capture antibody coated to the reaction well. The capture anibody binds the antigen of interest. In a second step a detection antibody is added that also binds to the antigen of interest. Said detection antibody is linked to a marker DNA, which can be amplified by PCR if the second antibody bound to the antigen and therefore is still present in the reaction well after washing. Due to the high sensitivity of the PCR low amounts of protein can be detected by this method.

The sensitivity of a ligand binding assay, in particular of immunoassays like ELISA or bead based binding assay, depends on a number of parameters including the affinity of the antibodies to the target molecule, the washing steps, the salt concentration in different solutions, the concentration of detergents and the type of detection system used, the blocking buffer, the duration of the incubation steps etc. Especially changing concentrations of solutions within the reaction well, e.g. during incubation steps, will impair the results and contribute to assay variability.

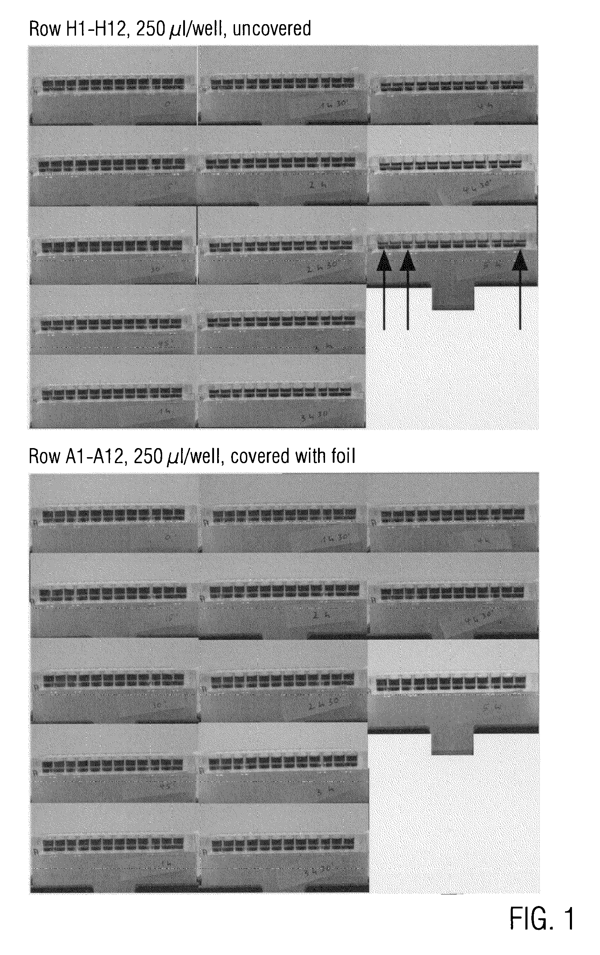

Interestingly, condensation and/or evaporative effects on a microtiter plate can vary from well to well. This has been shown by a test performed by the authors of the present invention. The results are shown in Fig. 1. For the test 250µl of a aqueous solution (distilled water colored with xylene and cyan blue) have been applied to two rows of wells (rows H and A) of a 96 microtiter plate (Greiner bio-one). Row H was left uncovered, whereas row A was covered with a foil. The plate was incubated at 37°C and 400 rpm in an Eppendorf Thermomixer Comfort and temperature inside the chamber was controlled continuously. Over a time period of 5 hours pictures were taken every 15 minutes. The results showed that the strongest condensation took place in wells H1, H12 and H3. After 5 hours about 30-40% of the solution was evaporated.

In summary this test demonstrates that due to unequal condensation in the different reaction wells the reaction condition within the different wells might vary strongly re-sulting in a high variability of the experimental data. The effect is also known to contribute as the inhomogeneity of results across a microtiterplate.

Furthermore, cross contaminations due to spillovers between the single reaction wells may occur if the wells are not covered or covered improperly.

This emphasizes the necessity to maintain the conditions stable and especially equal for every single well during the performance of the assay. Condensation and evaporative loss constitutes a major problem. During the incubation steps, which can be carried out at different temperatures (e.g. room temperature or higher), evaporation may lead to an increased salt concentration resulting in an increased probability of unspecific binding. Due to various incubation conditions (e.g. different room temperatures) condensation processes may vary leading to inconsistent and incorrect results. Typically, evaporation will lead to higher concentration of salts in solution which results in more unspecific binding of non-target molecules. This will increase background or lead to lower signal / noise ratios, finally to incorrect test results.

Experiments with diagnostic kits even showed that diagnostic tests performed with no covers or with rigid covers can even lead to false positive results due to unspecific binding caused by evaporative effects (ORTHO®HCV 3.0 ELISA Test System with Enhanced SA Ve, November 1, 1996).

In order to avoid problems like cross contamination or evaporation, the plates are usually sealed with a foil.

However, sealing with a foil comes along with a number of disadvantages. The foil is expensive and especially difficult to apply to the plates. Furthermore, despite the sealing with a foil, evaporation processes can occur and small amounts of the sample liquid might stick to the foil at the end of an incubation step requiring an additional centrifuging step. Due to the difficult handling of the foil, cross contaminations may arise when removing the foil from the plate due to shaking or quick movements of the plate or if the small drops hanging on the foil fall back in a well.

Another disadvantage of foil sealing appears when the assay should be performed automatically. While automated plate sealers exist, these are very expensive and difficult to integrate in a connected automated assay performance process.

Additionally, since after incubation the sample liquid is to be used for further experimentation (e.g. further incubation steps, washing steps or the detection step) the foil sealing has to be removed from the plate again or at least has to be pierced, requiring special tips and tools.

Similar problems come along with the usage of microplate caps or lids, which would also have to be removed either manually or mechanically.

One possibility to avoid cross contamination and/or evaporation processes has been described in the context of polymerase chain reaction (PCR) in 1986 and 1987 (EP 0236069 A2, cf. p. 48, I. 23; p. 51, II. 5-7; p. 54, II. 24-33). Thereby a layer of oil is applied to the top of the reaction mixture. However, this oil overlay has mostly been described in the beginning of the development of the PCR when the used thermocyclers were not equipped with a heated lid. Nowadays, almost all thermocyclers are equipped with heated lids to prevent condensation at the top of the reaction tube. Therefore, the use of an oil overlay is not required in most cases for performing PCR anymore. However, another crucial problem why people tried to find a new solution replacing the use of an oil overlay is that after finishing the PCR reaction, the sample liquid will be used for further experimentation steps (e.g. restriction digest or sequencing). However, due to the oil layer on top of the sample a transfer of small amounts of oil cannot be avoided. Therefore, the application of an oil layer on a PCR or different sensitive biochemical reaction has been replaced by the use of other sealing methods to avoid oil contaminations.

WO 2013/117606 discloses the usage of an oil layer in the context of performing PCR, branched DNA assay (bDNA), or sequencing (cf. p. 33, 2^{nd} paragraph) in order to prevent the liquid sample from coming into contact with air (cf. p. 32, last paragraph). Further, the authors of WO 2013/117606 mention that such an oil overlay can be added to the sample easily by any pipetting means (cf. p. 32, last paragraph and p. 34, last paragraph) and therefore, constitutes a practicable method for covering liquid samples automatically (cf. p. 33, 1^{st} paragraph). However, WO 2013/117606 only refers to DNA based assays like PCR, bDNA, or sequencing (cf. p. 33, 2^{nd} paragraph) as already described for PCR in 1987 in EP 0236069 A2 coming along with the problem of basically unavoidable oil contaminations in following experiments. WO 2013/117606 does not mention the usage of an oil layer for carrying out ligand binding assays, in particular not for solid-phase ligand binding assays like immunoassays.

Therefore, in order to avoid the above-mentioned problems for the performance of reliable ligand binding assays, it is the object of the present invention to provide a method for carrying out a ligand binding assay, preferably a solid-phase ligand binding assay, more preferably an immunoassay, in a way characterized in that:
- the risk of cross contamination is non-existent,
- the reaction conditions are homogeneous and stable over most of the time,
- condensation or evaporative loss is completely prevented,
- the method is not restricted to any temperature limitations, and/or
- the single steps of the assay are carried out in a way that the whole assay performance can easily be automated.

The object is solved by the method according to claim 1. Advantageous embodiments are defined in the corresponding subclaims.

According to the most general basic concept of the present invention, this object is obtained by the provision of a method for carrying out a ligand binding assay comprising the steps of filling at least one reaction well with a sample liquid and overlaying the sample liquid with an oil film as disclosed in claim 1.

Furthermore the present invention provides a device for an automated performance of a ligand binding assay comprising the following modules
(i) a pipetting module,
(ii) an incubator module, and
(iii) a reverse centrifuge module, and
(iv) a handling robot for moving the reaction well(s) to the respective modules in an automated manner.

A major advantage of the present invention is the usage of an oil layer overlaying the sample liquid when carrying out ligand binding assays, in particular solid-phase ligand binding assays like e.g. immunoassays, in particular ELISA or DNA capture assay. The oil layer should be applied at every experimental step, where condensation or an evaporative effect might occur, especially during incubation and/or detection steps. "Oil overlay" refers to a layer of oil, which in the end of its application procedure will completely cover the sample liquid and in cases where the reaction well is completely filled will cover the whole reaction well. The oil layer completely covers the liquid sample and/or reaction well(s) and basically no air is enclosed within the reaction well.

The oil layer together with other unbound reactants can easily be removed by washing, in particular by washing by means of reverse centrifuging. Therefore, residual amounts of oil causing problems in the further use of e.g. oil-overlayed PCR samples do not play a role for ligand binding assays, in particular for solid-phase ligand binding assays, since the whole oil can be removed easily. Thus, an impairment of any further experimental steps due to residual amounts of oil is basically excluded by the method according to the invention.

In a preferred embodiment of the present invention the ligand binding assay is a solid-phase ligand binding assay. The term "solid-phase ligand binding assay" includes any kind of assay wherein either one of a receptor molecule (e.g. antibody) or a target molecule (e.g. antigen) binds to or interacts with a solid phase (e.g. reaction well, (magnetic) bead, etc.). Also the binding or interaction of both a receptor molecule and a target molecule to the same solid phase at the same time or to several solid phases used during the assay is included.

Solid-phase ligand binding assays according to the invention are preferably immunoassays, more preferably ELISA, bead based ligand binding assays or DNA capture assays in all their various embodiments. These assays and the various ways of performing them are well known to a person skilled in the art.

The ligand binding assay according to the present invention can either be performed in one reaction well or in several reaction wells as can be found on multi- or micro-plates. The number of wells of those plates can range from six to 1536 and more. The generally used microplates have 24, 96, 384,or 1536 wells.

In a preferred embodiment of the present invention the maximum inner width of a reaction well is no more than 7 mm, preferably no more than 5 mm and more preferably no more than 4 mm.

"Incubation step" means any step requiring a certain time period to allow the forming of bindings between, e.g. ligand and receptor or coating molecules and reaction well etc, but also so called blocking steps performed to avoid unspecific bindings. Such time periods are necessary to allow the formation of bindings or complexes of reagents with each other or with any solid phase like the coating step, the step of adding e.g. the target molecule to the coated reaction well(s), the detection step comprising the addition of the enzyme or the substrate, etc.

The method of the present invention comprises the step of coating the reaction well(s) with either the desired receptor molecule, the desired target molecule or any other molecule which can be used for coating the plate depending on the experimental setup. In case of a sandwich ELISA monoclonal antibodies a preferred as receptor molecules. The amount of receptor or target molecule coated in the reaction well(s) can be varied depending on the experimental setup. For coating the reaction well(s) the reaction well or multiplate is filled with a solution containing the respective reagent and is then incubated for a time period of about half an hour and up to 2 to 10 hours. Depending on the antibody the incubation time for coating the reaction well(s) can possibly be shortened by incubating the reaction well(s) at a higher temperature than room temperature, e.g. at 37°C or higher. In order to avoid any evaporation at higher temperatures the sample liquid and/or reaction well(s) can be overlaid with an oil layer independent of the temperature the coating is carried out at.

The different incubation steps required for the various ligand binding assays are performed under the respective conditions, allowing e.g. the binding of the receptor molecules to the ligand molecules. Therefore, the temperature profile during incubation periods can be a constant temperature ranging from 4°C to 90°C. Of particular interest are temperatures around room temperature, 37°C and 50°C (especially for protein assays). Also a varying temperature profile depending on the assay and the used reagents can be applied. Even temperature profiles as used for PCR might be useful depending on the corresponding assay. Usually the amplification temperature profiles for PCR comprise a repeating sequence of a denaturation temperature, annealing temperature and an elongation temperature.

It can be assumed that also the time period needed for the formation of ligand-receptor complexes depends on the temperature applied. Thus, depending on the employed reagents reaction conditions can be adjusted accordingly. Since however, depending on the assay and the reagents, incubation time can vary from about 2 minutes up to 10 hours condensation and evaporative effects might be enormous. To avoid any of these undesired processes the sample liquid and/or the reaction well(s) is/are overlayed with an oil film.

Depending on the assay washing steps have to be performed between the single reaction steps. Washing can be carried out in many different ways like pouring out the plate manually and rinsing water over the plate at the tap. In a preferred embodiment of the present invention washing is performed by reverse centrifugation. Reverse centrifugation means that the opening(s) of the reaction well(s) are directed outwards, meaning away from the rotation axis. The rotation axis is, preferably parallel to the platform. Reversed centrifugation is performed as disclosed e.g. in WO 2013/117606 A1 or EP 13179437. Thereby the rotation axis is parallel to the platform of the sample carrier centrifuge. Preferably, the rotational axis is horizontal.

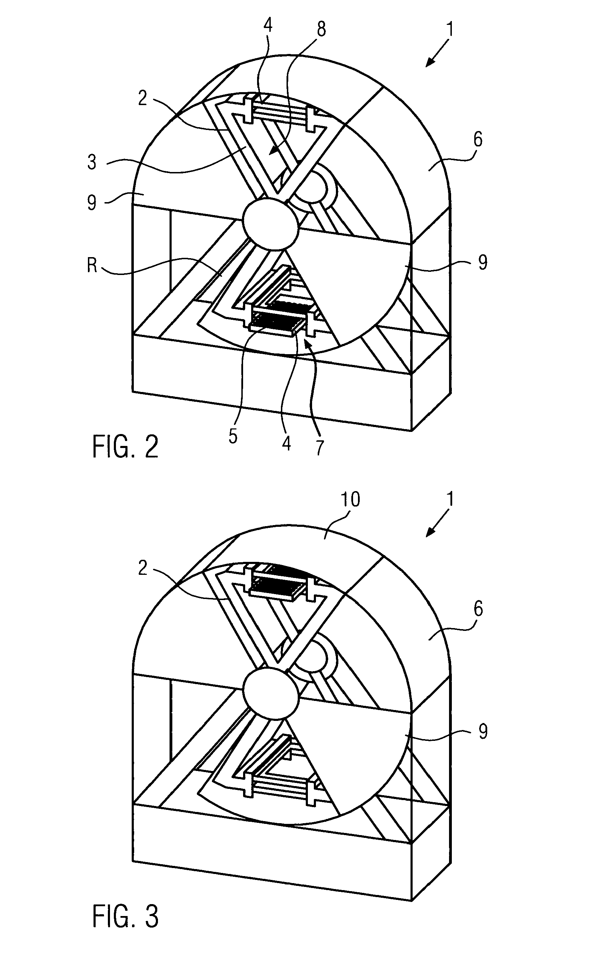

Fig. 2 shows schematically an embodiment of a centrifuge 1, which can be used for the method according to the invention. The centrifuge 1 comprises a rotor 2, which is rotatable mounted around the horizontal axis R. The rotor 2 comprises a framework 3 in the form of an X, wherein at the outer end of the framework 3 sample carrier receptacles 4 are provided. These receptacles 4 are embodied for taking up a microtiter plate 5.

The centrifuge 1 comprises a housing 6 with a lower opening 7 below the rotational axis R and an upper opening 8 above the rotational axis R. The openings 7, 8 can be closed by a rotatable door 9.

In Fig. 2, the centrifuge 1 is shown in a stage in which one receptacle 4 is placed on the bottom section and the other receptacle 4 is placed on the top section of the centrifuge. Both receptacles 4 are arranged horizontally in this position. A microtiter plate 5 can be loaded into the lower receptacle 4 by means of a horizontal, translational movement.

After loading the centrifuge, the door 9 closes the openings 7, 8 and the rotor is rotated around the rotation axis R.

As the centrifuge 1 is loaded or discharged by a horizontal translational movement of the microtiter plate 5, this centrifuge can be easily implemented in an automatic liquid handling system.

Fig. 3 shows the same centrifuge as in Fig. 2, however, the receptacle 4 placed at the upper portion of the centrifuge 1 is loaded with a microtiter plate. The vessels of the microtiter plate 5 are directed with their openings radially outwardly. By rotating the rotor 2, the content of the vessels of the microtiter plate 5 is splashed radially outwardly. This arrangement is used for washing the vessels of a microtiter plate 5. After loading the centrifuge with a microtiter plate 5, the rotor is rotated by 180° and stopped. All liquid content which does not adhere by means of surface tension is dropping out of the vessels into a bowl (not shown) placed below. Then the rotor is rotated with high rotational speed to expel all residual content from the vessels of the microtiter plate.

The housing 6 comprises at the top portion an automatically removable lid 10. The microtiter plate can be held directly below the upper portion of the housing 6 and then the lid 10 can be opened. With the pipetting means a washing solution can be introduced into the vessels of the microtiter plate and the rotation of the microtiter plate can be repeated with the washing solution. This process can be repeated several times.

Thus the centrifuge 1 can be used as a washing station for washing reaction vessels which are used for carrying out chemical and/or biological reactions.

Similar centrifuges have been disclosed in JP 2009-264927 and CN 102 175 855 A.

JP 2009-264927 A discloses a microplate treatment device comprising a rotating drum rotating around a horizontal rotation axis and having holding sections on a side surface of the rotating drum which each can hold a microplate. The drum is surrounded by a cover. The microplates can be placed in the drum so that the openings of the reaction vessels face to the outside or face to the inside of the drum.

From CN 102 175 855 A a washer for enzyme-labeled pates is known, comprising a rotating mechanism, a washing mechanism and a drainage mechanism. After the washing is completed, a centrifugal force, generated by continuous rotation, can throw off the water remaining in holes of the enzyme-labeled plates, so that a drying effect is realized, therefore the enzyme-level plates can be used at once after being washed.

By this method the content of the reaction well(s) can be removed easily, quick and in a reliable manner. Since according to the invention the sample liquid and/or the reaction well(s) is/are overlayed with an oil film no removal of any covering (e.g. foil or caps) is required but rather the well(s) can directly be centrifuged and emptied without leaving any residual amounts of oil in the well(s). After the well(s) has/have been emptied they can be filled with any washing solution or water (distilled, deionized or tap water) followed by another centrifuging step. This can be repeated several times (e.g. 2 to 5 times). If no washing is required the reaction well(s) can directly be filled with the desired reagent after being emptied.

The required step of detection to determine the result of the experiment depends on the performed assay. In cases when an ELISA is performed the detection is usually carried out by means of an enzyme-linked reporter molecule. The respective detection step might also require an incubation step whereby an oil layer can be applied on the reaction well(s) in order to maintain the reaction conditions constant and prevent any condensation or evaporative effects. Moreover, by applying an oil layer on the reaction well(s) and thereby preventing any condensation the detection step is not limited to any temperature but can rather be adjusted to the optimum temperature for e.g. the used enzyme as in the case for ELISA. By performing the detection step at the optimum temperature of e.g. an enzyme, the period for incubating the sample(s) can be shortened. Thus, the duration of the detection steps strongly depends on the way of detection and the conditions under which the detection is performed.

Thus, an oil overlay according to the present invention can be useful in order to decrease the duration of the single coating, incubation, washing, and/or detection steps by both its easy removal by, e.g. reverse centrifugation, and the absence of any temperature limitations since no condensation or evaporative effect can occur.

Another advantage of the oil overlay as disclosed herein can be observed for the performance of real-time PCR. During real-time PCR the amplified DNA is detected as the reaction progresses in "real time". Usually, for detection of DNA quantity fluorescent signals are measured. However, when measuring in reaction wells covered with foil the problem of stray light and quenching leading to decreased signal/noise ratios occurs. In a reaction well covered with oil this problem will not occur.

The oil layer can easily applied by any means of pipetting. There are basically two methods of applying the oil layer to the reaction well(s) as already described in WO 2013/117606, however, any other convenient way of application known to a person skilled in the art can be employed as well:

### Method a

A vessel can be initially provided with a layer of oil. The sample can be put into a vessel by means of pipetting. The vessel containing the sample on the layer of oil is centrifuged, whereby the sample is immersing the layer of oil so that after the centrifugation step the sample is covered by the layer of oil.

### Method b

A sample is put into a vessel. A layer of oil is put onto the sample. The vessel containing the sample and the layer of oil is centrifuged, wherein potential air between the sample and the layer of oil is expelled.

The oil used for the method according to the present invention is a mineral oil. Examples of mineral oils that can be used are mineral oils from Sigma Aldrich (EG number 232-455-8), Roth (EG number 232-455-8), Promega (EG number 232-384-2), VWR (EG number 232-455-8), or Genaxxon.

Moreover, by preheating the oil the desired temperature of the respective experimental step (e.g. incubation step) can be obtained faster and, therefore, the duration of the step to be carried out can be decreased. By using preheated oil the heat of the oil will be given off to the sample liquid and the desired temperature will be ensued faster. Therefore, the oil is preheated to the respective temperature desired for the experimental step to be carried out.

The amount of oil applied to the reaction well(s) depends mainly on the size of the well and the kind of employed oil. For 96 well plates about 20 to 50 µl of oil and for 384 well plates about 5 to 20 µl of oil are applied.

In summary, an oil overlay for ligand binding assays according to the present invention is accompanied by several advantages like
(a) easy handling/application of oil,
(b) easy adjustment of oil volumes,
(c) prevention of cross contamination,
(d) easy to remove by reverse centrifugation,
(e) optimized thermodynamics,
(f) no temperature limitations, and
(g) improved uniformity for all reaction wells.

A further crucial advantage of the method according to the invention is that by the advantages coming along with the usage of an oil overlay the whole process of a lig-and binding assay can be automated easily. Since ligand binding assays are often used for high throughput screenings automatized processes are highly preferred. It is obvious that in cases of high throughput screenings time can be a limiting factor. Therefore, any method allowing shortening the whole process is highly desirable as well. The method according to the invention even enables to combine both by providing a method according to the invention.

Additionally to the method, the present invention also provides a device for performing the method disclosed herein in a completely automated manner.

The device according to the invention comprises a pipetting module, which can be used with different tip sizes depending on the size of the employed reaction well(s). The pipetting module comprises at least one pipette and can be adjusted up to a number of pipettes as required depending on the number of reaction wells. Further, the pipetting module can be used to pipette all the different liquids required for the whole performing process of the assay. For example, the pipette module can be a robot arm. The device can further comprise a waste container for used tips which can automatically be removed from the pipette. These kind of pipetting robots are well known to a person skilled in the art.

Moreover, the device according to the invention comprises an incubator module which can be used to carry out any step of the assay at any desired temperature. Thus, the temperature of the incubator module can be adjusted as intended for the experiment for the respective time periods as needed. The incubator module can be either a separate module or can be integrated within any of the other modules of the device. The incubator module can be a thermocycler. Incubators, which can be used for the device according to the invention, are well known to a person skilled in the art. The device according to the invention further comprises a reverse centrifuge module as disclosed in WO 2013/117606 and/or EP 13179437 A1. Said reverse centrifuge module can additionally comprise the incubation module to perform any kind of incubation step directly inside of the centrifuge.

Further, the device according to the invention comprises a handling robot enabling the transport of the reaction well(s) or the plate from one module to the other, as needed, in an automated manner. The handling robot can be a separate module of the whole device or can be some connection between the modules transporting the reaction well(s) or plate from one to the other.

All the different modules together allow performing a complete ligand binding assay according to the invention in a fully automated way.

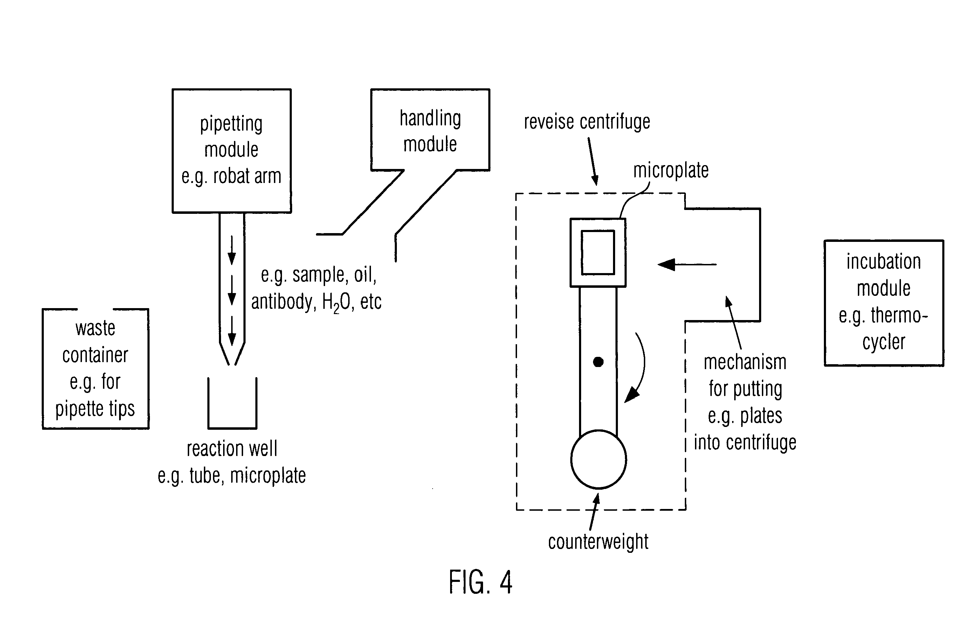

A scheme of the different modules of a device according to the invention as disclosed herein is shown in Fig. 4.

Additionally, the present invention provides control means suitable for controlling processes comprising a method disclosed herein. Respective control means are, e.g. microprocessors. Common control means required for the controlling of a method disclosed herein are well known to a person skilled in the art.

### Example

The following example is offered by way of illustration only and is by no means intended to limit the scope of the claimed invention.

Example 1 shows the comparison of an ELISA assay, wherein one microplate has been covered with an oil overlay (plate A) and one microplate was sealed with a foil (plate B) during the incubation steps.

An ELISA was performed using a human Ig G1 Ready Set Go kit (eBioscience, Affimetrix) with 96 well Corning Costar 9018 microplates.

The washing steps with washing buffer were carried out by means of reverse centrifugation (AusWasherMTP #57120) with 1000rpm for 10 seconds.

20 µl mineral oil HP50.2 (Roth) have been used for covering the wells on the respective plate (plate A).

4 plasma samples were obtained from two donors (indicated as #5 and #6) each.

### Assay procedure:

- coating: coating buffer and capture antibody over night at 4°C (alternatively at room temperature for 2 hours on a shaker at 400 rpm) with oil layer (plate A) or foil sealing (plate B),
- washing,
- blocking: blocking buffer for 2 hours at room temperature (alternatively over night at 4°C) with oil layer (plate A) or foil sealing (plate B),
- washing
- adding dilutions for standard curve and sample dilutions into the respective plate wells,
- incubation for 2 hours at room temperature with oil layer (plate A) or foil sealing (plate B),
- washing
- adding detection antibody and incubation for 1 hour at room temperature with oil layer (plate A) or foil sealing (plate B),
- washing
- adding substrate and incubation for 15 minutes at room temperature without any covering or sealing of the wells, before adding stop solution (eBioscience),
- detection at Victor Wallac^3 Multilable Counter (450nm)

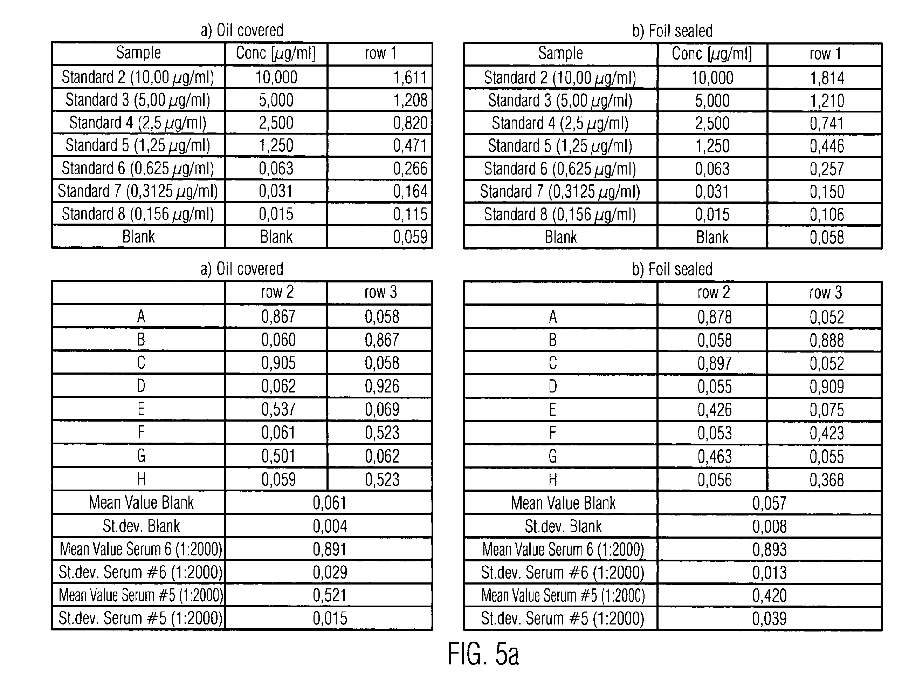

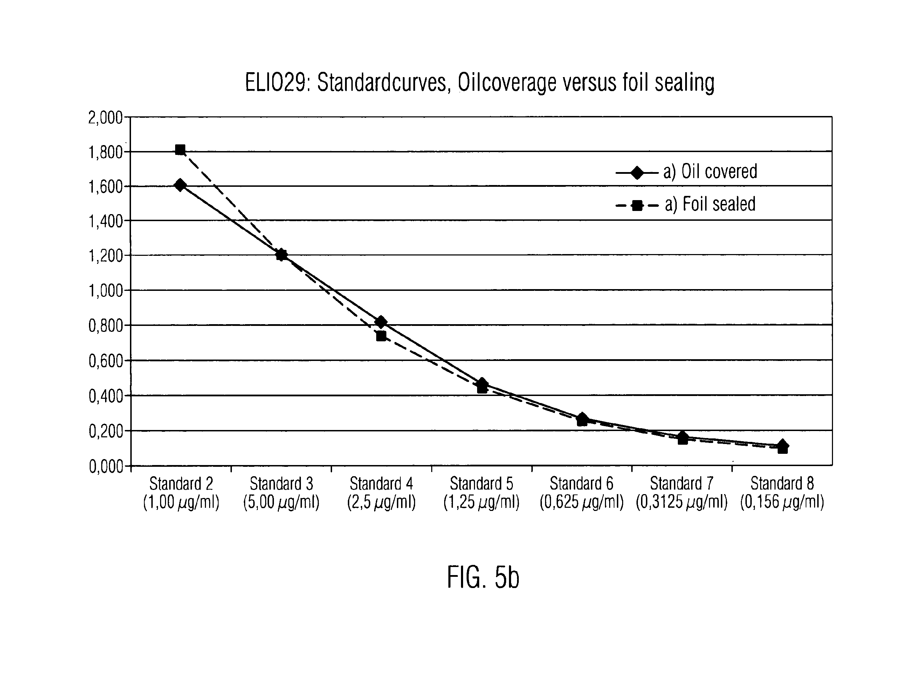

Figure 5a shows the values detected with the ELISA assay. The upper row shows the values detected for the standard curves, which were almost identical (see also Figure 5b).

The second row shows the values detected for the plasma samples (#6: rows A to D; #5: rows E to H; arranged staggered with blank samples in between).

Standard curves were almost identical on plate A and B (see Figure 5b).

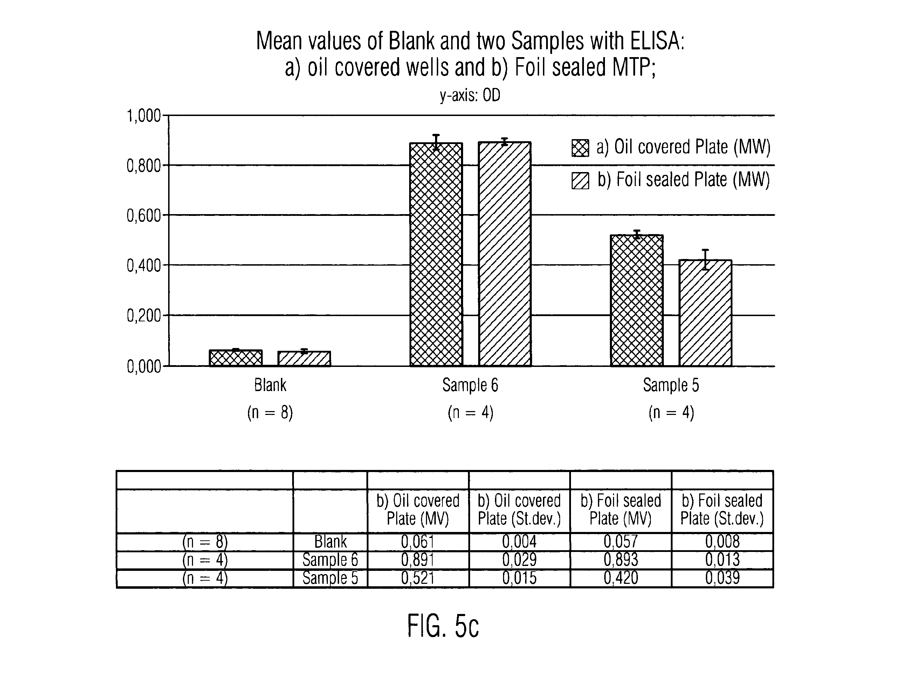

In Figure 5c the mean values (MV) of the blank, #6 and #5 samples on plate A and B are shown. For sample #6 no statistical difference could be observed for the two plates. However, the mean value of sample #5, which had an overall lower OD value, on plate A with the oil overlay was statistically higher. This demonstrates that the ELISA assay carried out using an oil overlay instead of foil sealing was more sensitive than the one with the foil sealed plate.

## Claims

**1.** Method for carrying out a ligand binding assay in at least one reaction well, comprising the steps of filling the at least one reaction well with a sample liquid and overlaying the sample liquid with an oil film.

**2.** Method according to claim 1, wherein
the ligand binding assay is a solid phase ligand binding assay, in particular, wherein the ligand binding assay is an immunoassay.

**3.** Method according to claim 1 or 2, wherein
the ligand binding assay is an enzyme-linked immunosorbent assay (ELISA), a DNA capture assay, or a bead based ligand binding assay.

**4.** Method according to one of the claims 1 to 3, comprising
a microplate with a plurality of reaction wells.

**5.** Method according to one of the claims 1 to 4, wherein
the at least one reaction well has a maximum inner width of not more than 7 mm, preferably not more than 5 mm and more preferably not more than 4 mm.

**4.** Method according to one of the claims 1 to 5, comprising
the step of coating the at least one reaction well with receptor molecules.

**5.** Method according to claim 4, wherein
the receptor molecules are monoclonal antibodies.

**6.** Method according to one of the claims 1 to 5, comprising
a step of incubating the filled reaction well(s) at a certain temperature.

**7.** Method according to claim 6, wherein
the temperature during incubation can be kept constant or can be varied over the time period.

**8.** Method according to one of the claims 1 to 7, comprising
washing of the at least one reaction well to remove non-bound material from the at least one reaction well.

**9.** Method according to claim 8, wherein
the washing is performed by means of reverse centrifuging.

**10.** Method according to one of the claims 1 to 9, wherein
the oil film is generated by pipetting a certain amount of oil in the respective reaction well(s) before filling the at least one reaction well with the sample liquid or after filling the at least one reaction well with the sample liquid.

**11.** Method according to one of the claims 1 to 10, wherein
the oil is mineral oil.

**12.** Method according to one of the claims 1 to 11, wherein the oil is preheated.

**13.** Device for an automated performance of the method according to claim 1 comprising the following modules
(i) a pipetting module,
(ii) an incubator module, and
(iii) a handling robot for moving the reaction well(s) to the respective modules in an automated manner.

**14.** Device according to claim 13, further comprising
a reverse centrifuge module.

**15.** Control means embodied for controlling a process comprising a method according to one of the claims 1-12.
